# EUROPEAN PATENT SPECIFICATION

(11) **EP 2 265 201 B1**
(45) Date of publication and mention of the grant of the patent: **03.10.2012**
(21) Application number: 09731953.7
(22) Date of filing: 30.03.2009
(51) Int. Cl.: A61B 17/70

(54) **SPINAL FIXATION SYSTEM**
WIRBELSÄULENFIXIERUNGSSYSTEM
SYSTÈME DE FIXATION SPINAL

(30) Priority: 19.04.2008 US 106286
(43) Date of publication of application: 29.12.2010
(73) Proprietor: Warsaw Orthopedic, Inc., Warsaw, IN 46581 (US)
(72) Inventor: TAYLOR, Harold, Sparr, Memphis TN 38112 (US); BAKER, Douglas, Neil, Collierville TN 38017 (US); CAPOTE, Marco, D., Boulder CO 80303 (US); CRANDALL, Dennis, G., M., D., Mesa AZ 85215 (US)
(74) Representative: Viering, Jentschura & Partner
(86) International application number: PCT/US2009/038800
(87) International publication number: WO 2009/129043

(56) References cited:
- WO-A-03/068088
- WO-A-2007/027934

## Description

The invention relates to a spinal fixation system according to the preamble of claim 1.

The present disclosure broadly concerns spinal fixation systems and generally relates to a multi-plane adjustment system involving a pivoting screw assembly. The system can be useful for correction of spinal injuries or deformities,

The present disclosure generally relates to a spinal fixation system useful for correction of spinal injuries or deformities. More specifically, but not exclusively, the present disclosure concerns spinal fixation systems allowing for pivotal motion of bone screws and lateral adjustment of spinal rod connectors relative to the bone screws, and providing locking mechanisms for locking the bone screws and connectors at desired positions. A spinal fixation system of the intially-mentioned type is known, for example, from WO 2007/027934 A2 or from WO 03/068088 A1.

In the realm of orthopedic surgery, it is well known to use implants to fix the position of bones. In this way, the healing of a broken bone can be promoted, and malformations or other injuries can be corrected. For example, in the field of spinal surgery, it is well known to place such implants into vertebrae for a number of reasons, including (a) correcting an abnormal curvature of the spine, including a scoliotic curvature, (b) to maintain appropriate spacing and provide support to broken or otherwise injured vertebrae, and (c) perform other therapies on the spinal column.

Implant and connection systems may include several pieces, which may be associated with only specific other pieces. Bone screws, hooks, clamps or other fixation devices can be connected or adjoined to a particular bone as a connection between the bone and the connection system, which can include a support and/or stabilizing member such as a spinal rod. In such a system, a series of two or more screws may be inserted into two or more vertebrae to be instrumented. A rod is then placed within or coupled to the screws, or is placed within a connecting device that links the rod and a screw, and the connection are tightened. In this way, a rigid supporting structure is fixed to the vertebrae, with the rod providing the support that promotes correction or healing of the vertebral malformation or injury by keeping the vertebrae in a particular position.

A spinal implant system or other similar system may have anchors that can be positioned at a number of angles with respect to the vertebrae or spinal rods. Such a feature allows easier placement of implant systems or correction of positioning of an implant system, in that the bone anchors need not be precisely positioned in angular relation with respect to the vertebrae or spinal rods. Rather, with a multi-axial capability, holes can be drilled in a bone at a convenient location and/or angle, for example, and screws can be inserted therein.

A need remains for spinal fixation systems allowing for multi-plane adjustment and which link elongated member(s) to fixation device(s) and allow for pivoting and other adjustment capabilities of the components of the system.

The invention provides a spinal implant system according to claim 1. Further embodiments of the invention are described in the dependent claims.

### BRIEF DESCRIPTION OF THE DRAWINGS

The example of Figs.3a-9 as said do not form embodiments of the invention, but help to understand aspects of the inventions.
FIG. 1 is a side view of an implant system according to one embodiment.
FIG. 2A is a front view of components of the implant system according to the embodiment shown in FIG. 1.
FIG. 2B is a side view of components of the implant system according to the embodiment shown in FIG. 1.
FIG. 3 is a perspective view of a component of the implant system according to the embodiment shown in FIG. 1.
FIG. 3A is a perspective view of a component of the implant system similar to the embodiment shown in FIG. 1.
FIG. 4 is a side view of another implant system
FIG. 5 is an exploded view of the implant system shown in FIG. 4.
FIG. 6 is a perspective view of components of the implant system shown in FIGS. 4 and 5.
FIG. 7 is a side view of another implant system
FIG. 8 is a partial exploded view of the implant system shown in FIG. 7.
FIG. 9 is front view of the implant system shown in FIGS. 7 and 8.

### DESCRIPTION OF THE ILLUSTRATED EMBODIMENTS

For the purpose of promoting an understanding of the principles of the disclosure, reference will now be made to the embodiments illustrated in the drawings and specific language will be used to describe the same. It will nevertheless be understood that no limitation of the scope of the claims is thereby intended, such alterations and further modifications in the illustrated device, and such further applications of the principles of the disclosure as illustrated therein, being contemplated as would normally occur to one skilled in the art to which the disclosure relates.

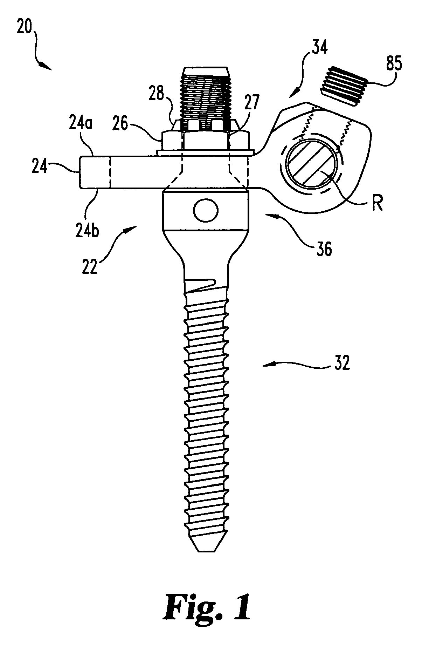

Referring generally to FIGS. 1-3, there is shown an embodiment of a spinal fixation system 20. System 20 generally includes a fixation device or anchor (e.g. a pivoting bone screw or bolt 22), a connector 24 and a nut 26 in that embodiment. Connector 24, having a top surface 24a and a bottom surface 24b, can connect an elongate member, such as a spinal rod, a bar, or other such orthopedic construct, to anchor 22. The elongate member may have any of a number of desired lengths. Bone screw 22 includes a bone engaging portion 32, a pivoting post portion 34 and a hinge portion 36 between the bone engaging portion 32 and pivoting post portion 34. Hinge portion 36 permits pivotal motion of post portion 34 relative to bone engaging portion 32.

In certain embodiments, bone engaging portion 32 can be threaded to engage a bone structure, such as a vertebral body, and solidly anchor screw 22 to the bone structure. In such embodiments, bone engaging portion 32 can include coarse threads readily adapted for solid fixation within the cancellous bone of a vertebral body and can terminate in a tapered tip to assist in the gradual engagement and advancement of the threads into the vertebral body. In alternative embodiments, it should be appreciated that bone engaging portion 32 can have a variety of configurations and/or can be a hook or other such appropriate bone engaging structure. Post portion 34 can also be threaded to engage nut 26 or other such threaded items. In certain embodiments, post portion 34 includes machine threads upon which a surgeon may attach an item, such as a clamp. Nut 26 defines a threaded inner hole 27 to threadedly couple with post portion 34. Nut 26 is operable to lock pivoting post portion 34 at a desired angular position relative to bone engaging portion 32. Turning and tightening nut 26 thereby locks connector 24 at the same desired angular position as post portion 34. In certain embodiments, nut 26 can include locking tabs 28 to further secure and lock system 20 at desired positions.

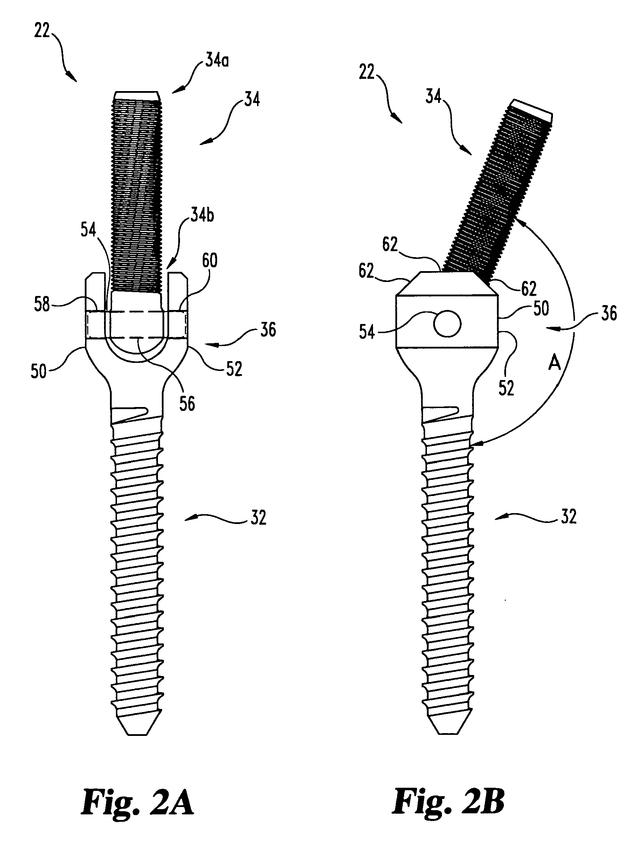

FIG. 2A is a front view of anchor 22 according to the embodiment shown in FIG. 1. Post portion 34 includes a proximal end 34a and a distal end 34b, with distal end 34b being operably coupled with hinge portion 36. Hinge portion 36 of screw 22 can include extensions 50 and 52. In certain embodiments, extensions 50 and 52 are generally parallel and extend upward from bone engaging portion 32. Extensions 50 and 52 are spaced apart to generally define a channel to receive distal end 34b of post portion 34. The width of the space between extensions 50 and 52 may be larger than the diameter of post portion 34. Hinge portion 36 of screw 22 can also include a hinge pin 54. In certain embodiments, post portion 34 rotates about hinge pin 54 relative to bone engaging portion 32. Additionally, post portion 34 includes an aperture 56 and extensions 50 and 52 include apertures 58 and 60, respectively, to receive hinge pin 54. In certain embodiments, hinge pin 54 can be received and maintained in apertures 56, 58, and 60 in an interference fit relationship. During assembly, pin 54 can be inserted through one aperture 58 or 60, through aperture 56 in post portion 34, and through the other of aperture 58 or 60.

FIG. 2B is a side view of anchor 22 of the embodiment shown in FIG. 1. As illustrated, post portion 34 can pivot about hinge 54 relative to bone engaging portion 32. In the illustrated embodiment, post portion 34 is pivoted to a position such that an angle A exists between post portion 34 and bone engaging portion 32. Extensions 50 and 52 of hinge portion 36 can include three generally straight segments 62 to contact connector 24. In other embodiments, straight segments 62 can number more or less than three. Additionally, in other embodiments, extensions 50 and 52 can include generally curved convex or conical upper surfaces to contact connector 24. Straight segments 62 are configured to contact bottom surface 24b of connector 24. Straight segments 62 can correspond to angular position(s) of post portion 34 relative to bone engaging portion 32.

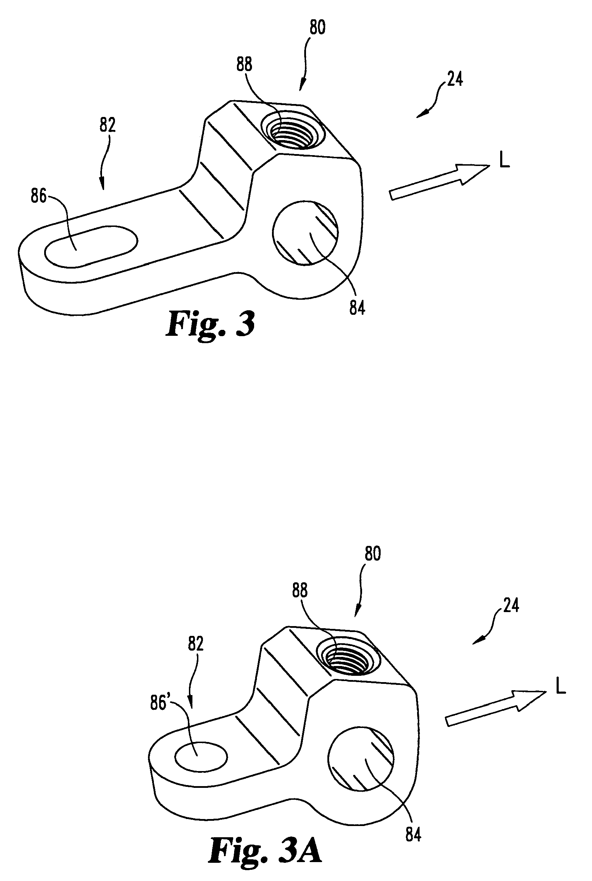

FIG. 3 is a perspective view of connector 24 according to the embodiment shown in FIG. 1. In the illustrated embodiment, connector 24 extends along a longitudinal axis L and includes a elongated member engaging portion 80 and an anchor engaging portion 82. Portion 80 defines a channel 84 configured for receipt of an elongate member, such as a spinal rod. In certain embodiments, channel 84 is generally perpendicular to longitudinal axis L. Portion 82 defines an elongate slot 86 in this embodiment, which is configured in this embodiment for receipt of post portion 34 of bone screw 22. In certain embodiments, slot 86 extends generally along longitudinal axis L. FIG. 3A shows connector 24 having a substantially circular hole 86' in an anchor engaging portion 82 that is somewhat shorter than the portion 82 in FIG. 3.

Additionally, portion 80 can define a through hole 88 in communication with channel 84 configured to receive a retaining member, such as a set screw 85. In certain embodiments, hole 88 can include internal threaded portion(s) to be threadedly coupled with the retaining member to secure an elongate member in channel 84. In such embodiments, the internal threaded portions can include reverse angle threads, i.e. a thread in which the forward face points as disclosed in U.S. Patent 6,296,642.

In certain embodiments, retaining member 85 is a generally cylindrical set screw with external threads, but may alternatively be another type of holding or locking mechanism. However, it should be appreciated that the elongate member can be secured in channel 84 by other appropriate methods. In certain embodiments, channel 84 may be non-circular in shape to better allow for a retaining member, such as a set screw, to tightly retain an elongate member in channel 84. It should be appreciated that channel 84 can be configured and shaped differently as would generally occur to one skilled in the art.

In certain embodiments, slot 86 includes a sufficient length along longitudinal axis L to allow for adjustment of post portion 34 and positioning of post portion 34 at any one of a plurality of positions along slot 86. The available positioning of post portion 34 at a plurality of positions along slot 86 thereby allows for the relative positioning of an elongate member received in channel 84 at a plurality of positions relative to bone screw 22. Nut 26 is operable to lock pivoting post portion 34 at a desired position along slot 86.

Referring generally to FIGS. 1-3, the operation and use of system 20 will be described with reference to a surgical procedure involving a section of spine. It will be appreciated that other uses of system 20 in other surgical procedures can be made.

To treat the condition or injury of the patient, the surgeon obtains access to the surgical site in a manner well known in the art, e.g. through incision and retraction of tissues. Once access to the surgical site has been obtained, e.g. via an opening such as a midline incision above the affected area, with tissue being resected laterally to the transverse process, or by other surgical procedure, the surgeon may connect one or more implants, such as bone screws, to adjacent or nearby vertebrae that require compression or distraction in order to relieve or improve their condition. For example, pilot holes in vertebrae, e.g. in pedicles, may be made, and anchors (e.g. screws 22) may be inserted into or otherwise connected to two or more vertebrae. In one embodiment, once an appropriate access to a surgical site is obtained, system 20 can be inserted to the surgical site, and may be placed in a desired position at or adjacent certain vertebrae. For example, screw 22 may be inserted into a bony structure, such as a vertebra, at a desired position. Threaded portion 32 of each such screw 22 can be threaded into bone to a desired depth, and connector 24 may be placed on screw 22 so that portion 34 extends through slot 86.

An elongate member (e.g. rod R) is placed in channel 84 of connector 24 either before or after connection of connector 24 to screw 22 or placement of system 20 near vertebrae. Retaining member 85 can be received in hole 88 of connector 24 to loosely hold elongate member R therein, while allowing translational and rotational adjustibility. Elongate member R can later be received in another connecting assembly with a bone screw or other implant inserted into another vertebra to secure a section of vertebrae.

In certain embodiments, a user of system 20 can pivot post portion 34 to a desired angular position relative to bone engaging portion 32. In the illustrated embodiment, bottom surface 24b of connector 24 can contact one or more of straight segments 62 of extensions 50 and 52 corresponding to a desired angular position of post portion 34. In alternative embodiments, connector 24 engages extensions 50 and 52 in other manners as would occur to one skilled in the art. Additionally, a user can position post portion 34 at a desired location along slot 86 of connector 24. Prior to locking, connector 24 can pivot with respect to portion 32 of anchor 22, or can be moved around an axis of portion 34 of anchor 22. Connector 24 can also be translated along slot 86 with respect to screw 22. Connector 24 can also be pivoted with respect to or translated along elongated member R.

After multi-plane adjustment of system 20, it can be locked to fix the relative positions of elongated member R, screw 22 and connector 24. In certain embodiments, nut 26 can engage post portion 34 by threading nut 26 down post portion 34 to thereby lock system 20 at the desired position by clamping connector 24 to hinge portion 36 of bone screw 22. In certain embodiments, locking tabs 28 can be used to further engage post portion 24 and lock system 20 and the desired positions. Either before or after locking of system 20, a user can insert portion 32 of anchor 22 in a vertebral body and can insert elongate member R in channel 84. Set screw or other retaining member 85 is tightened in hole 88 to securely hold member R in connector 24. System 20 may generally be assembled prior to use in a surgical procedure. However, it should be appreciated that system 20 can be assembled during the surgical procedure.

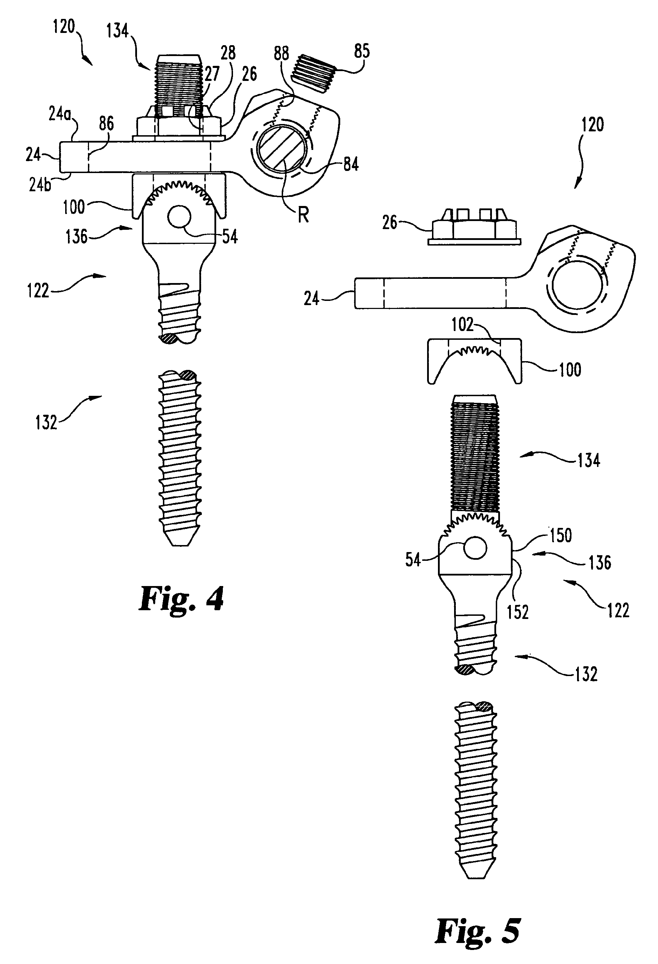

FIG. 4 illustrates another system 120 where like reference numerals refer to like features previously discussed. System 120 generally includes a pivoting anchor such as bone screw 122, connector 24 and nut 26. Connector 24 connects an elongate member, such as a spinal rod R, to screw 122. System 120 can further include a locking piece 100 positionable below connector 24 adjacent bottom surface 24b.

FIG. 5 is an exploded view of system 120. As illustrated, locking piece 100 defines a hole 102 configured to receive the post portion of screw 122. Screw 122 can include bone engaging portion 132, pivoting post portion 134 and hinge portion 136 between portion 132 and pivoting post portion 134. Similar to anchor 22, hinge portion 136 of screw 122 permits pivotal motion of post portion 134 relative to engaging portion 132. In certain embodiments, bone engaging portion 132 can be threaded to engage a vertebra. Additionally, post portion 134 can be threaded to engage nut 126 or other such items.

As illustrated, hinge portion 136 of bone screw 122 includes extensions 150 and 152. In certain systems extentions 150 and 152 are generally parallel and extend upward from bone engaging portion 132. Additionally, hinge portion 136 can include a hinge pin 54 about which pivoting post portion 134 rotates relative to bone engaging portion 132. Similar to screw 22, post portion 134 and extensions 150 and 152 can include holes to receive hinge pin 54.

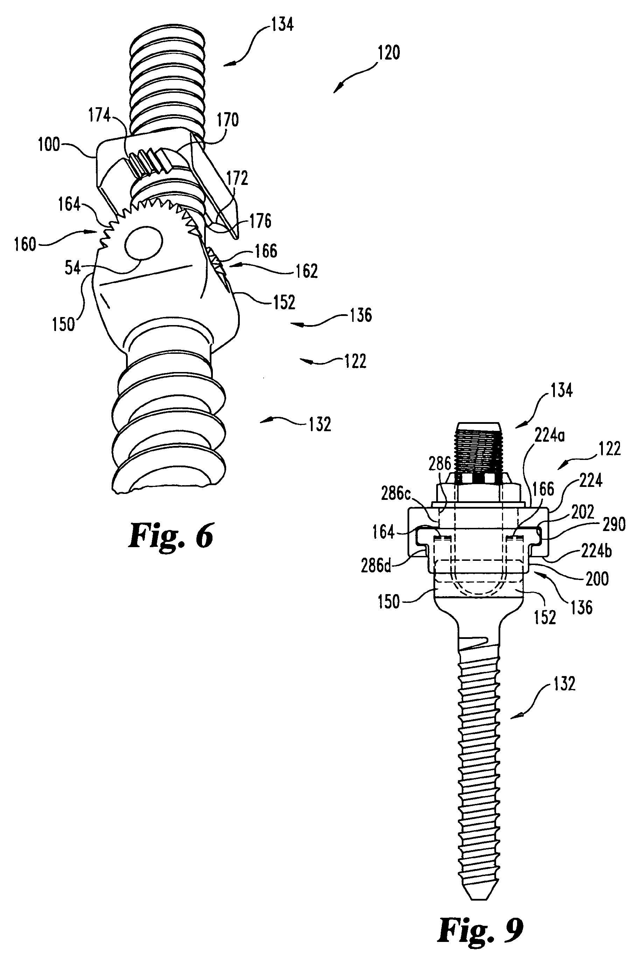

FIG. 6 illustrates select components of system 120, including extensions 150 and 152 and locking piece 100. Extensions 150 and 152 generally include upper convex surfaces 160 and 162, respectively, having splines 164 and 166, respectively. A bottom surface of locking piece 100 includes two generally parallel concave surfaces 170 and 172 having splines 174 and 176, respectively, to engage splines 164 and 166 on extensions 150 and 152 at a plurality of positions. The engagement of the sets of splines at various positions corresponds to various angular positions of post portion 134 relative to bone engaging portion 132. In alternative systems the splines on the extensions and the locking piece are absent, with generally curved concave and convex surfaces of the extensions and the locking piece being in engagement.

Referring generally to FIGS. 4-6, the operation and use of system 120 will be described with reference to a surgical procedure involving a section of spine. It will be appreciated that other uses of system 120 in other surgical procedures can be made. The surgeon obtains access to the surgical site as previously descried in greater detail. In one embodiment, once an appropriate access to a surgical site is obtained, system 120 can be inserted to the surgical site, and may be placed in a desired position at or adjacent certain vertebrae. Thereafter, screw 122 is inserted into a bony structure, such as a vertebra, at a desired position. Threaded engaging portion 132 can be threaded into bone to a desired depth.

An elongate member (e.g. rod R) is placed in channel 84 of connector 24 either before or after connection of connector 24 to anchor 122 or placement of system 120 near the vertebrae. A retaining member (e.g. set screw 85) can be received in hole 88 of connector 24 to loosely hold elongate member R therein, while allowing translational and rotational adjustability. Elongate member R can later be received in another connecting assembly with a bone screw or other implant inserted into another vertebra to secure a section of vertebrae.

A user of system 120 can pivot post portion 134 to a desired angular position relative to bone engaging portion 132. In the illustrated embodiment, bottom surface 24b of connector 24 contacts an upper surface of locking piece 100. Additionally, splines on locking piece 100 contact splines on extensions 150 and 152 at a desired position corresponding to a desired angular position of post portion 134. In alternative systems, locking piece 100 engages extensions 150 and 152 in other manners. Additionally, similar to system 20, a user can position post portion 134 at a desired location along slot 86 of connector 24. Connector 24 can pivot with respect to portion 132 and along with portion 134, and can translate along slot 86 with respect to anchor 122. Connector 24 can also be pivoted and/or translated with respect to elongated member R.

After multi-plane adjustment of system 120, it can be locked so as to fix elongated member R, connector 24 and screw 122 with respect to each other. In certain systems, engagement of nut 26 to post portion 134 by threading nut 26 down post portion 134 operates to lock system 120 at the desired position by clamping connector 24 between nut 26 and locking piece 100, thereby clamping locking piece 100 to hinge portion 136 of bone screw 122. Additionally, similar to system 120, either before or after locking of system 120, a user can insert bone engaging portion 132 in a vertebra and can insert an elongate member R in channel 84 of connector 24. A retaining member (e.g. set screw 85) may be tightened in hole 88 to securely hold elongated member R to connector 24. System 120 may generally be assembled prior to use in a surgical procedure. However, it should be appreciated that system 120 can be assembled during the surgical procedure.

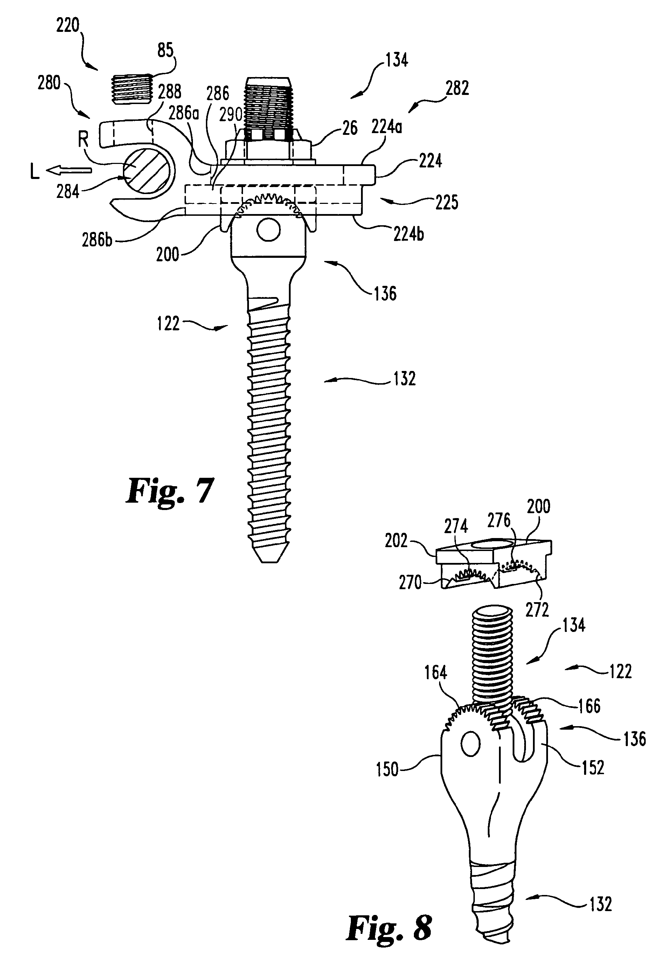

FIG. 7 illustrates a system 220 according to another system where like reference numerals refer to like features previously discussed. System 220 generally includes a pivoting anchor such as bone screw 122, a connector 224 and nut 26. Connector 224 is operable to connect an elongate member, such as a spinal rod R, to bone screw 122 and includes a top surface 224a and a bottom surface 224b. System 220 further includes a locking piece 200 positionable within or along connector 224. In the illustrated system connector 224 includes a recessed area 225 to permit easier insertion of locking piece 200 within connector 224.

As illustrated connector 224 can extend along a longitudinal axis L and can include an elongated member engaging portion 280 and an anchor engaging portion 282. Portion 280 defines a substantially C-shaped channel 284 configured for receipt of an elongate member, such as a spinal rod R. In certain systems, channel 284 is generally perpendicular to longitudinal axis L. In the illustrated system channel 284 is a side loading spinal rod channel. However, it should be appreciated that channel 284 can be sized and/or configured differently as would occur to one skilled in the art. Additionally, rod engaging portion 280 can define a through hole 288 in communication with channel 284 configured to receive a retaining member 285, such as a set screw. The retaining member is operable to secure an elongate member in channel 284.

Bone screw engaging portion 282 defines an elongate slot 286 configured for receipt of post portion 134 of bone screw 122. In certain embodiments, slot 286 extends generally along or parallel to longitudinal axis L. Bone screw engaging portion 282 can also defme longitudinal grooves 290 adjacent slot 286 configured to engage locking piece 200 (see FIG. 9). In the illustrated system grooves 290 extend generally along or parallel to longitudinal axis L, with one groove 290 on each side of slot 286. In certain systems longitudinal grooves 290 run substantially the entire length of each longitudinal side of slot 286.

Similar to slot 86, slot 286 includes a sufficient length along longitudinal axis L to allow for adjustment of post portion 134 and positioning of post portion 134 at any one of a plurality of positions along slot 286. In certain systems slot 286 includes an upper section 286a having a first length and a lower section 286b having a second length, with upper section 286a adjacent top surface 224a of connector 224 and lower section 286b adjacent bottom surface 224b of connector 224. Additionally, in certain systems lower section 286b includes a greater length along longitudinal axis L than upper section 286a to increase the plurality of positions post portion 134 can occupy in slot 286. The variable positioning of post portion 134 in slot 286 allows for the positioning of an elongate member received in channel 284 at a plurality of distances or positions relative to bone screw 122. In certain systems, nut 26 is also operable to lock pivoting post portion 134 at the desired position within slot 286.

FIG. 8 is a partial exploded view of system 220. In certain systems locking piece 200 is integral with connector 224 (see FIG. 7). Locking piece 200 can be positioned in slot 286 and can include extensions, such as fingers 202, to slidably engage channels 290. Similar to locking piece 100, a bottom surface of locking piece 200 includes two generally parallel concave surfaces 270 and 272 having splines 274 and 276, respectively, to engage splines 164 and 166 on extensions 150 and 152 at a plurality of positions. In alternative systems the splines are absent and the concave and convex surfaces of locking piece 200 and extensions 150 and 152 are slidably engaged. The engagement of sets of splines at various positions corresponds to the various angular positions of post portion 134 relative to bone engaging portion 132.

FIG. 9 illustrates a front view of system 220. As illustrated, locking piece 200 can include one or more fingers 202 on opposite sides of locking piece 200 to be slidably received in grooves 290. In the illustrated system, fingers 202 are generally rectangular in cross-sectional shape. However, it should be appreciated that fingers 202 can be otherwise sized and configured. Slot 286 includes an upper width section 286c adjacent top surface 224a of connector 224 and a lower width section 286d adjacent bottom surface 224b of connector 224. In certain embodiments, lower section 226d includes a width greater than upper section 226c to accommodate loading and translation of locking piece 200 in slot 286. Locking piece 200 can be positioned at a plurality of positions along slot 286 thereby positioning post portion 134 at one of a plurality of positions along slot 286.

Referring generally to FIGS. 7-9, the operation and use of system 220 will be described with reference to a surgical procedure involving a section of spine. It will be appreciated that other uses of system 220 in other surgical procedures can be made. The surgeon obtains access to the surgical site as previously descried in greater detail. In one embodiment, once an appropriate access to a surgical site is obtained, system 220 can be inserted to the surgical site, and may be placed in a desired position at or adjacent certain vertebrae. For example, screw 122 may be inserted into a bony structure, such as a vertebra, at a desired position. Threaded portion 132 of each such anchor 122 can be threaded into bone to a desired depth.

An elongate member (e.g. rod R) is placed in channel 84 of connector 224 either before or after connection of connector 224 to screw 122 or placement of system 220 near the vertebrae. A retaining member (e.g. set screw 85) can be received in hole 288 of connector 224 to loosely hold elongate member R therein, while allowing translational and rotational adjustability. Elongate member R can later be received in another connecting assembly with a bone screw or other implant inserted into another vertebra to secure a section of vertebrae.

In certain systems, a user of system 220 can pivot post portion 134 to a desired angular position relative to bone engaging portion 132. In certain embodiments, splines on locking piece 200 can contact splines on extensions 150 and 152 at a desired position corresponding to a desired angular position of post portion 134. In alternative systems locking piece 200 engages extensions 150 and 152 in other manners as would occur to one skilled in the art. Connector 224 can pivot with respect to portion 132 and along with portion 134, and can translate along slot 286 with respect to anchor 122. Connector 224 can also be pivoted and/or translated with respect to elongated member R.

Similar to systems 20 and 120, a user of system 220 can position post portion 134 at a desired location along slot 286 of connector 224 via the sliding engagement of locking piece 200 within connector 224. The positioning of post portion 134 along slot 286 corresponds to the positioning of locking piece 200 along slot 286 by the sliding of fingers 202 along grooves 290.

After multi-plane adjustment of system 220, it can be locked so that elongate member R, connector 224 and anchor 122 are fixed with respect to each other. In certain systems, the engagement of nut 26 to post portion 134 by threading nut 26 down post portion 134 thereby locks system 20 at the desired positions by clamping connector 224 and locking piece 200 to hinge portion 136 of bone screw 122. Additionally, similar to systems 20 and 120, a user of system 220 can insert bone engaging portion 132 in a vertebral body and can insert an elongate member in side loading channel 284. System 220 may generally be assembled prior to use in a surgical procedure. However, it should be appreciated that system 220 can be assembled during the surgical procedure.

The parts, features and steps discussed above may be interchanged with each other or among embodiments. Accordingly, anchor 22 may be used with system 220, as one example. The various components of systems 20, 120, and 220 are composed of biocompatible materials such as titanium, stainless steel, certain ceramics or plastics, or others.

## Claims

1. A spinal implant system (20), comprising:
a fixation element having a threaded bone engaging portion (32), a pivoting portion (34), and a hinge portion (36) between said bone engaging portion (32) and said pivoting portion (34) to permit pivotal motion of said pivoting portion (34) relative to said bone engaging portion (32), whereby said pivoting portion (34) is positionable in any one of a plurality of angular positions relative to said bone engaging portion (32);
a connector body (24) having an elongate member engaging portion (80) and a fixation element engaging portion (82), wherein said elongate member engaging portion (80) defines a channel (84) configured for receipt of an elongate member (R), and said fixation element engaging portion (82) defines an elongate slot (86) configured for receipt of said pivoting portion (34) of said fixation element, wherein said pivoting portion (34) is positionable in any one of a plurality of positions along said elongate slot (86); and
a compression member (26) adapted to engage said pivoting portion (34) of said fixation element and press against said connector body (24), to thereby lock said pivoting portion (34) at a desired angular position relative to said bone engaging portion (32) and lock said connector body (24) to said fixation element, wherein
said hinge portion (34) includes a hinge member (54) and two lateral extensions (50, 52) extending from said bone engaging portion (32), each of said extensions (50, 52) and said pivoting portion (34) of said fixation element defining a hole (58, 60) for passage of said hinge member (54), **characterized in that**
each of said extensions (50, 52) includes top surface having two or more straight segments (62) to contact a lower surface (24b) of said fixation element engaging portion (82)
of said connector body (24), said straight segments corresponding to angular positions of said pivoting portion (34) relative to the bone engaging portion (32).

2. The spinal implant system (20) of claim 1, wherein said connector body (24) has a longitudinal axis and said elongate slot (86) extends parallel to said longitudinal axis, wherein said elongate slot (86) includes a length along said longitudinal axis to allow for lateral adjustment of said connector body (24) relative to said fixation element, wherein said connector body (24) is positionable adjacent said hinge portion (36) of said fixation element when said pivoting portion (34) is received in said elongate slot (86).

3. The spinal implant system (20) of claim 1, wherein said pivoting portion (34) is threaded, said compression member (26) is a nut, and said nut includes an inner threaded surface defining a hole, and said nut is adapted to be threaded onto said pivoting portion (34) and down onto said fixation element engaging portion (82) of said connector body, whereby said threaded pivoting portion is received in said hole.

4. The spinal implant system (20) of claim 1, comprising a hole defined by said elongate member engaging portion in communication with said channel configured to receive a retaining member to secure an elongate member in said channel.

## Patentansprüche

1. Ein Wirbelsäulenimplantatsystem (20), aufweisend:
ein Befestigungselement, aufweisend einen Gewinde-Knocheneingriffsabschnitt (32), einen Schwenkabschnitt (34) und einen Gelenkabschnitt (36) zwischen dem Knocheneingriffsabschnitt (32) und dem Schwenkabschnitt (34), um eine Schwenkbewegung des Schwenkabschnitts (34) bezüglich des Knocheneingriffsabschnitts (32) zu ermöglichen, wodurch der Schwenkabschnitt (34) bezüglich des Knocheneingriffsabschnitts (32) in irgendeiner aus einer Mehrzahl von Winkelpositionen positionierbar ist;
einen Verbindungskörper (24), der einen Langgestrecktes-Element-Eingriffsabschnitt (80) und einen Befestigungselement-Eingriffsabschnitt (82) aufweist, wobei der Langgestrecktes-Element-Eingriffsabschnitt (80) einen Kanal (84) definiert, der zum Aufnehmen eines langgestreckten Elements (R) eingerichtet ist, und wobei der Befestigungselement-Eingriffsabschnitt (82) einen langgestreckten Schlitz (86) definiert, der zum Aufnehmen des Schwenkabschnitts (34) des Befestigungselements eingerichtet ist, wobei der Schwenkabschnitt (34) entlang dem langgestreckten Schlitz (86) in irgendeiner aus einer Mehrzahl von Positionen positionierbar ist; und ein Kompressionselement (26), das angepasst ist, um mit dem Schwenkabschnitt (34) des Befestigungselements in Eingriff zu sein und gegen den Verbindungskörper (24) zu drücken, um dadurch den Schwenkabschnitt (34) bezüglich des Knocheneingriffsabschnitts (32) in einer gewünschten Winkelposition zu verriegeln und den Verbindungskörper (24) an dem Befestigungselement zu verriegeln, wobei
der Gelenkabschnitt (34) ein Gelenkelement (54) und zwei seitliche Verlängerungen (50, 52) aufweist, die sich von dem Knocheneingriffsabschnitt (32) aus erstrecken, wobei jede/jeder von den Verlängerungen (50, 52) und dem Schwenkabschnitt (34) des Befestigungselements ein Loch (58, 60) zum Hindurchpassieren des Gelenkelements (54) definiert, **dadurch gekennzeichnet, dass**
jede von den Verlängerungen (50, 52) eine obere Fläche aufweist, die zwei oder mehr gerade Segmente (62) aufweist, um eine untere Fläche (24b) des Befestigungselement-Eingriffsabschnitts (82) des Verbindungskörpers (24) zu kontaktieren, wobei die geraden Segmente bezüglich des Knocheneingriffsabschnitts (32) mit Winkelpositionen des Schwenkabschnitts (34) korrespondieren.

2. Das Wirbelsäulenimplantatsystem (20) gemäß Anspruch 1, wobei der Verbindungskörper (24) eine Längsachse aufweist und sich der langgestreckte Schlitz (86) parallel zu der Längsachse erstreckt, wobei der langgestreckte Schlitz (86) entlang der Längsachse eine Länge hat, um ein seitliches Verstellen des Verbindungskörpers (24) bezüglich des Befestigungselements zu ermöglichen, wobei der Verbindungskörper (24) benachbart zu dem Gelenkabschnitt (36) des Befestigungselements positionierbar ist, wenn der Schwenkabschnitt (34) in dem langgestreckten Schlitz (86) aufgenommen ist.

3. Das Wirbelsäulenimplantatsystem (20) gemäß Anspruch 1, wobei der Schwenkabschnitt (34) mit einem Gewinde versehen ist, das Kompressionselement (26) eine Mutter ist und die Mutter eine ein Loch definierende Innengewindefläche aufweist, und wobei die Mutter angepasst ist, auf den Schwenkabschnitt (34) und hinunter auf den
Befestigungselement-Eingriffsabschnitt (82) des Verbindungskörpers geschraubt zu sein, wodurch der Gewinde-Schwenkabschnitt in dem Loch aufgenommen ist.

4. Das Wirbelsäulenimplantatsystem (20) gemäß Anspruch 1, aufweisend ein durch den Langgestrecktes-Element-Eingriffsabschnitt definiertes Loch in Kommunikation mit dem Kanal, das eingerichtet ist, ein Halteelement aufzunehmen, um ein langgestrecktes Element in dem Kanal zu sichern.

## Revendications

1. Système d'implant rachidien (20), comprenant :
un élément de fixation comportant une partie d'engagement osseux filetée (32), une partie pivotante (34) et une partie charnière (36) entre ladite partie d'engagement osseux (32) et ladite partie pivotante (34) afin de permettre un mouvement de pivot de ladite partie pivotante (34) par rapport à ladite partie d'engagement osseux (32), moyennant quoi ladite partie pivotante (34) peut être positionnée dans l'une quelconque d'une pluralité de positions angulaires par rapport à ladite partie d'engagement osseux (32) ;
un corps formant connecteur (24) comportant une partie d'engagement d'un élément allongé (80) et une partie d'engagement d'un élément de fixation (82), où ladite partie d'engagement d'un élément allongé (80) définit un canal (84) configuré pour recevoir un élément allongé (R), et ladite partie d'engagement d'un élément de fixation (82) définit une fente allongée (86) configurée pour recevoir ladite partie pivotante (34) dudit élément de fixation, où ladite partie pivotante (34) peut être positionnée dans l'une quelconque d'une pluralité de positions le long de ladite fente allongée (86) ; et un élément de compression (26) adapté pour s'engager dans ladite partie pivotante (34) dudit élément de fixation et exercer une pression contre ledit corps formant connecteur (24), afin de verrouiller ladite partie pivotante (34) selon une position angulaire souhaitée par rapport à ladite partie d'engagement osseux (32) et verrouiller ledit corps formant connecteur (34) audit élément de fixation, où ladite partie charnière (34) comprend un élément charnière (54) et deux extensions latérales (50, 52) qui s'étendent à partie de ladite partie d'engagement osseux (32), chacune desdites extensions (50, 52) et ladite partie pivotante (34) dudit élément de fixation définissant un orifice (58, 60) pour le passage dudit élément charnière (54), **caractérisé en ce que**
chacune desdites extensions (50, 52) comprend une surface supérieure comportant deux segments droits (62) ou davantage afin de créer un contact avec une surface inférieure (24b) de ladite partie d'engagement d'un élément de fixation (82) dudit corps formant connecteur (24), lesdits segments droits correspondant à des positions angulaires de ladite partie pivotante (34) par rapport à ladite partie d'engagement osseux (32),

2. Système d'implant rachidien (20) selon la revendication 1, dans lequel ledit corps formant
connecteur (24) possède un axe longitudinal et ladite fente allongée (86) s'étend parallèlement audit axe longitudinal, où ladite fente allongée (86) comprend une longueur le long dudit axe longitudinal afin de permettre un ajustement latéral dudit corps formant connecteur (24) par rapport audit élément de fixation, où ledit corps formant connecteur (24) peut être positionné de manière adjacente à ladite partie charnière (36) dudit élément de fixation lorsque ladite partie pivotante (34) est reçue dans ladite fente allongée (86).

3. Système d'implant rachidien (20) selon la revendication 1, dans lequel ladite partie pivotante (34) est filetée, ledit élément de compression (26) est un écrou, et ledit écrou comprend une surface interne filetée définissant un orifice, et ledit écrou est adapté pour être vissé sur ladite partie pivotante (34), et jusqu'à ladite partie d'engagement d'un élément de fixation (82) dudit corps formant connecteur, moyennant quoi ladite partie pivotante filetée est reçue dans ledit orifice.

4. Système d'implant rachidien (20) selon la revendication 1, comprenant un orifice défini par ladite partie d'engagement d'un élément allongé en communication avec ledit canal configuré pour recevoir un élément de retenue afin de maintenir un élément allongé dans ledit canal.
